# EUROPEAN PATENT SPECIFICATION

(11) **EP 4 274 466 B1**
(45) Date of publication and mention of the grant of the patent: **02.04.2025**
(21) Application number: 22737119.2
(22) Date of filing: 06.01.2022
(51) Int. Cl.: A61B 1/31

(54) **SYSTEM FOR TREATING HEMORRHOIDS**
SYSTEM ZUR BEHANDLUNG VON HÄMORRHOIDEN
SYSTÈME DE TRAITEMENT D'HÉMORROÏDES

(30) Priority: 06.01.2021 US 202163134183 P
(43) Date of publication of application: 15.11.2023
(73) Proprietor: Minimax Medical (Nanjing) Co., Limited, Jiangning High-tech Industrial Park Nanjing City Jiangsu 211112 (CN)
(72) Inventor: EUM, Jay J., Irvine, CA 92602 (US)
(74) Representative: Swea IP Law AB
(86) International application number: PCT/US2022/011481
(87) International publication number: WO 2022/150504

(56) References cited:
- WO-A1-2004/021874
- WO-A1-2020/217223
- WO-A2-2005/020931
- CN-A- 104 116 548
- CN-U- 202 052 190
- US-A- 4 898 169
- US-A1- 2008 262 511
- US-A1- 2008 262 511
- US-A1- 2009 005 647
- US-A1- 2009 318 940
- US-A1- 2010 130 857
- US-A1- 2018 125 350

## Description

### BACKGROUND OF THE INVENTION

### 1. Field of the Invention

The present invention relates to a system for treating hemorrhoids according to claim 1.

### 2. Description of the Related Art

Hemorrhoids are swollen blood vessels in the rectum. There are two basic types of hemorrhoids: internal and external.

Internal hemorrhoids are swollen and inflamed veins far up in the rectum. Internal hemorrhoids cannot be seen or felt and usually are not painful due to the paucity in nerve endings in the upper portion of the rectum. While internal hemorrhoids are most commonly manifested by anal bleeding, they may prolapse or protrude outside the anal sphincter. Usually, prolapsed internal hemorrhoids may be gently pushed back into place in the rectum.

External hemorrhoids are swollen blood vessels in the anus and are usually manifested by pain as well as bleeding. When external hemorrhoids prolapse or protrude from the anal sphincter, blood clots sometimes form, causing an extremely painful condition known as thrombosis. While they usually disappear by themselves within about a week, thrombosed hemorrhoids may be removed by a physician or may be treated with a pain- reducing medication to reduce the pain.

It is believed that hemorrhoids are caused by the exertion of abdominal pressure on rectal veins, causing the veins to swell and become irritated. The abdominal pressure may be caused by a variety of factors and conditions, including obesity, pregnancy, prolonged standing or sitting, liver disease, straining during bowel movements, coughing, sneezing, vomiting, or holding the breath during physical activity. Hemorrhoids are largely preventable by the adoption of a high-fiber diet. On the other hand, person whose diet consists largely of low-fiber and processed foods tend to run the highest risk of developing hemorrhoids. Furthermore, inadequate fluid intake can contribute to the development of hemorrhoids by causing the development of hard stools, which irritate and inflame the rectal veins.

About half of population living in the United States will be afflicted with hemorrhoids at some point during their lives. Hemorrhoids most often strike people between the ages of 20 and 50. Some evidence indicates that "weak" veins, which are most susceptible to developing hemorrhoids, are inherited.

The present invention relates to applying heat energy by forms of conductive, resistive heating, and Radiofrequency heating. More particularly, the present invention relates to utilizing an innovative anoscope to position, monitor, and treat hemorrhoids. Lastly, the present invention utilized various probes to apply heat energy.

US 2008/262511 A1 discloses an anoscope for inspection and/or surgery. The anoscope includes a tubular body having a distal end, a proximal end, and a longitudinal axis defined there between, where the tubular body includes at least one elongated slot. The anoscope also includes an insert removably attached to the at least one elongated slot in the tubular body. The insert includes an elongated slot with a width that is smaller than that of the at least one elongated slot in the tubular body.

### SUMMARY OF THE INVENTION

According to the present invention a system for treating hemorrhoids includes an anoscope assembly and a treatment probe. The anoscope assembly includes an elongated anoscope body and a sliding element. The elongated anoscope body has a distal end and a proximal end. The anoscope body is configured to be insertable into the rectum of a patient. The elongated anoscope body includes a main portion and a proximal portion. The main portion has a longitudinal slot configured to function as a window opening to trap hemorrhoids. The proximal portion depends proximally from the main portion. The proximal portion has handles thereon configured to be manipulated by a user so as to adjust the cross-sectional area of the proximal portion. The sliding element is configured to slide through the proximal portion and through the longitudinal slot when the cross-sectional area of the proximal portion is sufficiently increased by a user by manipulation of the handles. This provides access and secure placement of the sliding element within the longitudinal slot. The treatment probe operably connected to the anoscope assembly is configured to provide energy to a hemorrhoid during treatment. Said proximal portion defining a broken away ring type structure, said broken away ring type structure separates from the main portion by a cutaway radial slot and remains integrally connected to the main portion by an integrally connecting portion.

In an aspect of the present disclosure, not falling within the scope of the claims, there is a method for treating hemorrhoids that includes providing an anoscope assembly, providing a treatment probe, inserting the anoscope assembly into the rectum, inserting the treatment probe into the rectum, performing a treatment by utilizing the treatment probe, and removing the anoscope assembly. The anoscope assembly includes an elongated anoscope body and a sliding element. The elongated anoscope body has a distal end and a proximal end. The anoscope body is configured to be insertable into the rectum of a patient. The elongated anoscope body includes a main portion and a proximal portion. The main portion has a longitudinal slot configured to function as a window opening to trap hemorrhoids. The proximal portion depends proximally from the main portion. The proximal portion has handles thereon configured to be manipulated by a user so as adjust the cross-sectional area of the proximal portion. The sliding element is configured to slide through the proximal portion and through the longitudinal slot when the cross-sectional area of the proximal portion is sufficiently increased by a user by manipulation of the handles. This provides access and secure placement of the sliding element within the longitudinal slot. The treatment probe operably connected to the anoscope assembly is configured to provide energy to a hemorrhoid during treatment

In one aspect, the ablating apparatus for use in treating a hemorrhoid of the present invention includes a combination of an anoscope, ablation catheter/paddle probe, dilator, and ground pad. The anoscope incorporates an adjustable window on the side of the scope body, which is positionable within an orifice. The window is closed during the insertion of the scope but is opened to receive the hemorrhoid tissue and applies clamping pressure onto the tissue. The adjustable window could further incorporate a return path for the RF signal and/or a temperature measuring element to monitor the tissue temperature. The ablation catheter includes an ablating element for treating the tissue and a temperature measuring element for monitoring the ablating element during treatment. The dilator includes a hollow lancing shaft within which an ablation catheter is situated. The dilator sheath (a hollow lancing shaft) has a central passage for the drainage of fluids. The ablation catheter, the dilator, and the anoscope is controlled or monitored by a control unit that displays the temperatures from the temperature measuring elements of the ablating apparatus and supplies energy to the ablating element of the catheter.

The ablating element may use any number of modalities that are suitable for ablating a hemorrhoidal tissue. The ablating element may be a contact catheter that can be used without the dilator. The ablating element may be a heating element that does not require electricity to flow from the ablation element to the body. Examples of ablation elements include but are not limited to a resistive heater, a radiofrequency electrode, a laser heating fiber, and a microwave antenna probe. If it is a resistive heater, it may be, for example, a resistive wire heater, semi-conductor material heater, or resistive sheath heater. The energy source may be, for example, direct current, alternating current, or radiofrequency current. The ablating element may be a cooling element, such as a cryosurgical device.

The ablating apparatus can be used with a multi-functional control system for controlling the overall ablative process, including, for example, monitoring the temperature of the ablating device and surrounding tissue, timing the ablation, controlling the energy level to the ablating device, and controlling the energy path from the ablation catheter to anoscope and/or to ground pad.

Other objects, advantages, and novel features will become apparent from the following detailed description of the invention when considered in conjunction with the accompanying drawings.

### BRIEF DESCRIPTION OF THE DRAWINGS

Figure 1 is a perspective view of an embodiment of the dilator assembly of the system for treating hemorrhoids of the present invention.
Figure 1A is a sectional view of the dilator assembly taken along line 1A-1A of Figure 1.
Figure 2 is a view of an embodiment of the dilator sheath assembly of the system for treating hemorrhoids of the present invention.
Figure 2A is a sectional view of the dilator sheath assembly taken along line 2A-2A of Figure 2.
Figure 3 is a perspective view of an embodiment of an ablation element (probe) of the system for treating hemorrhoids of the present invention, embodied as an ablation catheter.
Figure 3A is a sectional view of the ablation catheter, taken along line 3A-3A of Figure 3.
Figure 3B is a schematic view of the ablation catheter assembly connectors.
Figure 4 is a perspective view of an embodiment of the paddle ablation probe assembly.
Figure 4A is a sectional view of paddle ablation probe.
Figure 4B is a sectional view of another embodiment of paddle ablation probe.
Figure 4C is a view of paddle probe connectors.
Figure 5 is a view of the anoscope assembly.
Figure 5A is a sectional view of the anoscope body.
Figure 5B is another view of the anoscope body.
Figure 5C is a view of the anoscope sliding window.
Figure 5D is a sectional view of sliding window connectors.
Figure 6A-6G are views of procedural steps for using an ablation catheter.
Figure 7A- 7E are views of procedural steps for using a paddle ablation probe.
Figure 8 is a block diagram of the overall system.
Figure 9A is a block diagram of the current path of one embodiment.
Figure 9B is a block diagram of the current path of another embodiment.
Figure 9C is a block diagram of the current path of yet another embodiment.
Figure 10 is a front perspective view of another embodiment of the ablation probe (catheter).
Figure 10A is a rear perspective view of the ablation probe of Figure 10.
Figure 10B is a perspective view of a thin paddle probe.
Figure 11 is a perspective view of a preferred embodiment of the anoscope assembly in a nearly closed position.
Figure 11A is an exploded perspective view of the anoscope assembly of Figure 11.
Figure 11B is a view taken along line 11B-11B of Figure 11.

### DETAILED DESCRIPTION OF THE INVENTION

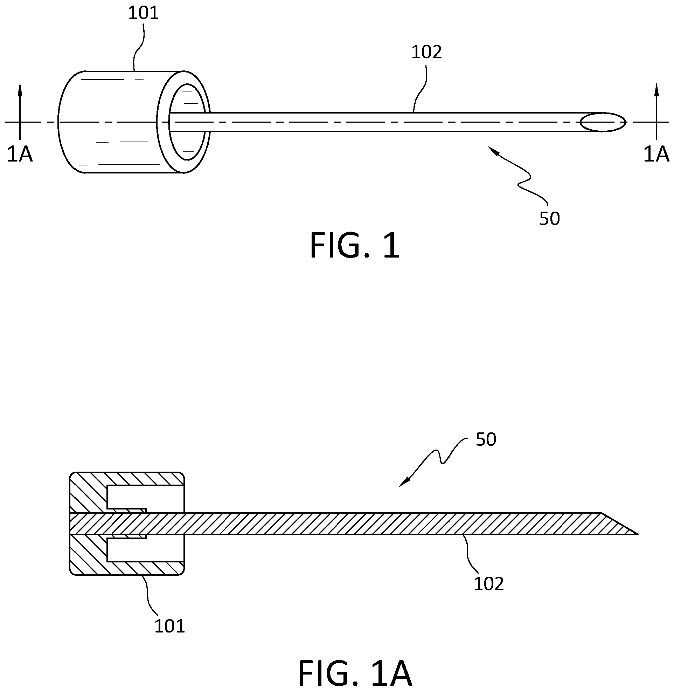

Referring to Figures 1 and 1A, dilator assembly 50 includes a dilator connector 101 connected to a dilator needle 102.

The length and diameter of the dilator needle 102 are configured with dilator sheath 104 such that the needle 102 has to fit inside of the sheath 104 and the needle 102 has to extend beyond the end of the sheath 104 by the length of the ablation zone formed by ablation catheter 52. The dilator may be formed of rigid materials, for example, but not limited to stainless steel and titanium. The tip of the dilator needle 102 has a smaller diameter than the diameter of the proximal end of the catheter for penetration into tissues. Examples of the tips are, for example, but not limited to, trocar and needlepoint tips.

Dilator connector 101 is designed to mate with sheath connector 103. Dilator connector 101 is attached to dilator 102. The dilator connector 101 may be formed with a dielectric material, for example, but not limited to polycarbonate, ABS, and Nylon, etc.

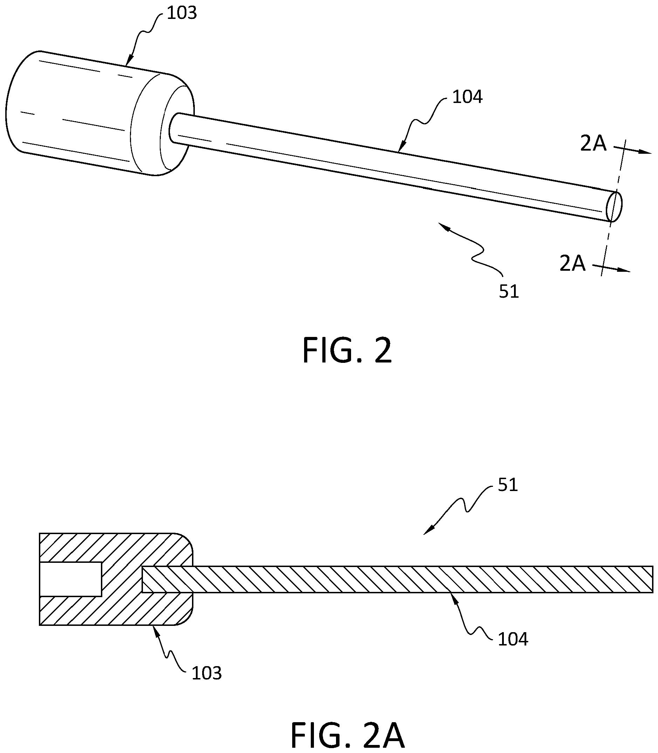

Referring now to Figures 2 and 2A, dilator sheath assembly 51 includes dilator sheath connector 103 and dilator sheath 104. There is a channel formed in the middle of the dilator connector 103, which extends along the length of the dilator sheath 104, allows dilator 50 to be positioned inside of the sheath assembly 51. The dilator connector 101 mates with dilator sheath connector 103. The dilator connector 103 is designed to mate with the connector of syringe 129. The dilator sheath 104 may be formed with high dielectric and rigid material, for example, but not limited to mylar and PTFE. The dilator sheath connector 103 may be formed with a dielectric material, for example, but not limited to polycarbonate, ABS, and Nylon, etc.

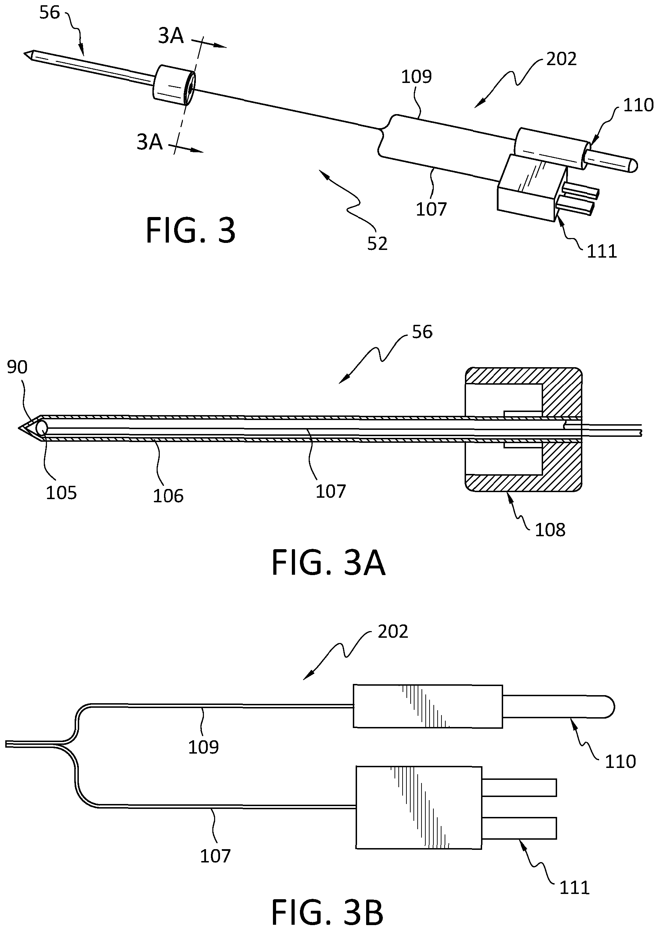

Referring to Figures 3, 3A, and 3B, ablation catheter assembly 52 includes and ablation catheter element 56. Ablation catheter element 56 includes ablation catheter temperature sensor 105, ablation catheter sheath 106, ablation catheter temperature sensing cable 107, and ablation catheter connector 108. the ablation catheter assembly 52 also includes ablation catheter connectors including ablation catheter power cable 109, an ablation catheter power connector 110, and an ablation catheter temperature sensing connector 111, cable 107, cable 109, connector 110, and connector 111, collectively define a connector system designated generally as 202.

The ablation catheter temperature sensor 105 is preferably attached near the tip 90 of the inside of the ablation catheter sheath 106. The temperature sensor 105 is used to monitor the maximum temperature reached at the ablation zone of the ablation catheter 52. For the safety of the ablation, it is ideal, not only to measure the temperature at the extent of the targeted hemorhoid 128, but also to measure the temperature at the energy source to prevent the over treatment of the tissues adjacent to the catheter tip 90 while the other tissues of hemorrhoid away from the tip 90 has not reached the temperature. The temperature sensor 105 can be utilized using different temperature measuring modalities, for example, but not limited to thermocouples, thermistors, and RTDs (Resistance Thermometer Detectors).

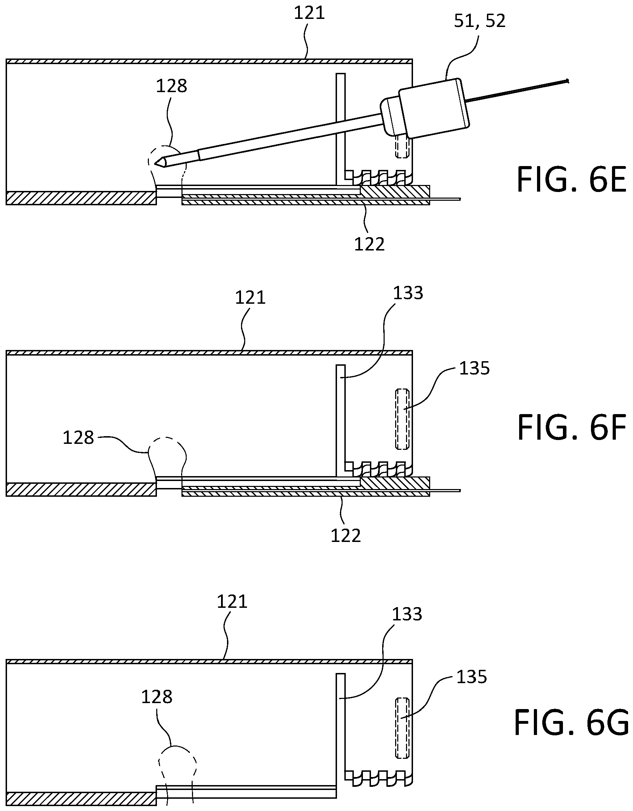

The ablation catheter sheath 106 is attached to the ablation catheter connector 108 and extends therefrom. The ablation catheter sheath 106 fits inside of the dilator sheath element 104 and extends beyond the end of the dilator sheath element 104. The outside surface of the ablation catheter sheath 106 touches hemorrhoid 128 (see, for example, Figure 6E). For one of the embodiments that utilizes RF power flow from the ablation catheter to the hemorrhoid tissue to deliver heat, the ablation catheter sheath is formed from electrically conductive and biocompatible materials, for example, but not limited to stainless steel and titanium. Preferably, the tip of the ablation catheter sheath has a smaller diameter than the diameter of the proximal end of the catheter. Examples of the tips are, for example, but not limited to, trocar and needlepoint tips. If the user utilizes a dilator to penetrate the hemorrhoid before inserting the ablation catheter element 56 into hemorrhoid, the tip 90 of the ablation catheter sheath 106 can be blunt.

One end of the ablation catheter temperature sensing cable 107 is attached to the temperature sensor 105. The other end of the temperature sensing cable is attached to the temperature sensor connector 111. The temperature sensing cable 107 is positioned inside of the catheter probe sheath 106 for the portion that extends along the ablation probe sheath. If a thermocouple is used for temperature sensor 105, temperature sensing cable 107 and temperature sensor 105 is the same assembly of a thermocouple junction wire. If the temperature sensor 105 is a thermistor or RTD, etc., the temperature sensing cable comprises a pair of electrical wires insulated from each other.

The ablation catheter connector 108 is connected to the ablation catheter sheath 106 and configured to mate with the dilator sheath assembly 51. The ablation catheter connector 108 can be formed with dielectric materials, for example, but not limited to polycarbonate, ABS, and Nylon, etc.

One end of the ablation catheter power cable 109 is connected to the ablation catheter power connector 110. For the embodiment of utilizing RF energy, the other end of the catheter power connector 109 is connected to the ablation catheter sheath 106. The ablation catheter power cable 109 is positioned inside of the catheter probe sheath 106 for the portion that extends along the ablation probe sheath. The ablation catheter power cable is a single or multiple conduction cables.

In an embodiment utilizing thermocouple wires as the ablation catheter temperature sensor 105, temperature sensing connector 111 is a thermocouple connector that matches the compound of the thermocouple wire used. In an embodiment using a thermistor or RTD as temperature sensor 105, temperature sensing connector 111 is an electrical connector. One end of the temperature connector is attached to the thermocouple cable 107, and the other end plugs into the system 55.

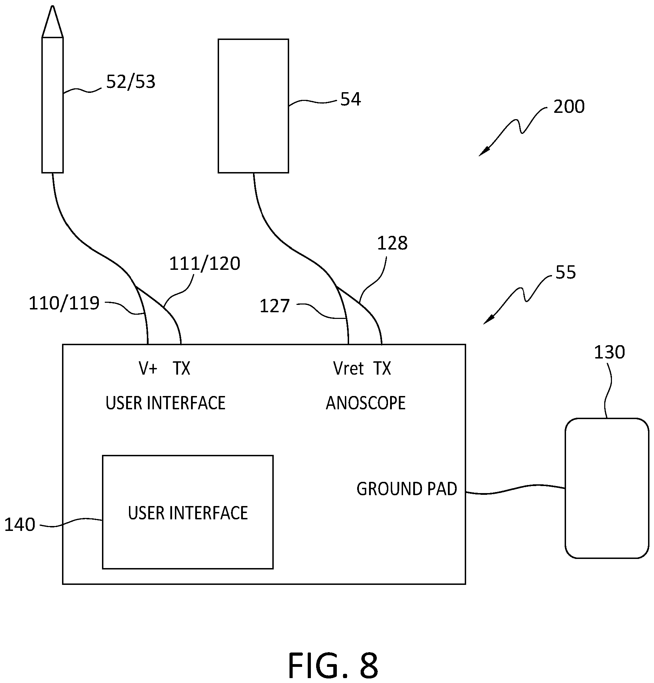

One end of the ablation catheter power connector 110 is connected to the ablation catheter power cable 109, and the other end of the power connector plugs into the system 55(see Figure 8). The ablation catheter power connector 110 has a portion that is electrically conductive that mates to electrically conductive material located inside of the system 55. The conductive material in the power connector 110 is preferably not exposed to the user. Outside housing of the power connector 110 can be formed with dielectric materials, for example, but not limited to polycarbonate, ABS, and Nylon, etc.

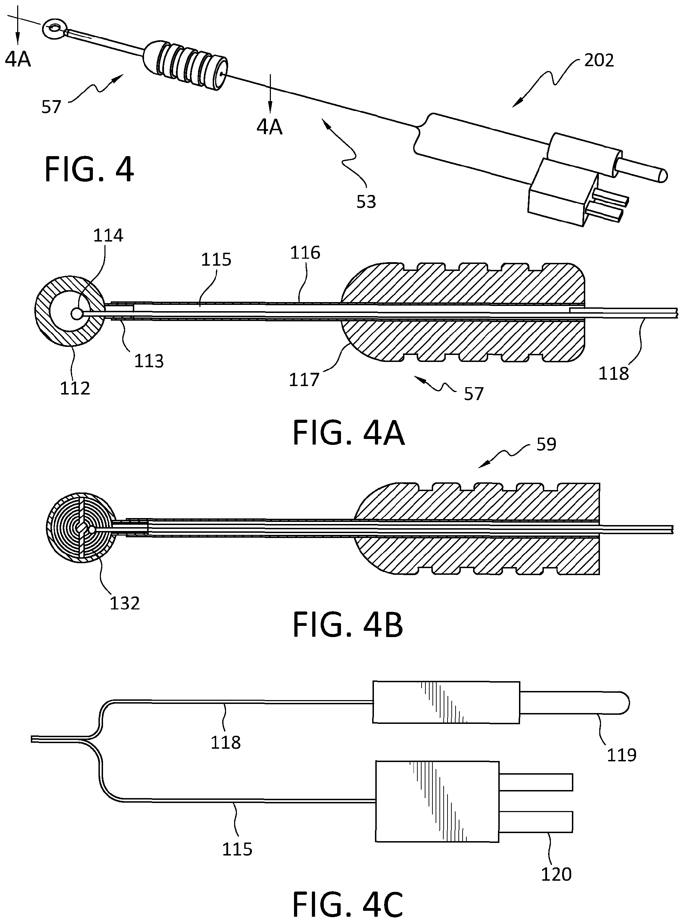

Referring to Figures 4, 4A, 4B, and 4C, a paddle ablation probe assembly 53 includes a paddle ablation probe element 57. Paddle ablation probe element 57 includes paddle ablation disk 112, paddle ablation probe sheath 113, paddle ablation probe temperature sensor 114, paddle ablation probe sensor cable 115, paddle ablation probe insulation sheath 116, paddle ablation probe handle 117. The paddle ablation probe assembly 53 also includes a paddle ablation power cable 118, paddle ablation probe power connector 119, and paddle ablation probe temperature connector 120. In another embodiment (see Figure 4B) the paddle ablation probe 59 includes resistive material 132 to apply heat energy to hemorrhoid.

The paddle ablation disk 112 is attached to the paddle ablation probe sheath 113. The paddle ablation disk 112 is designed to fit inside of the anoscope body 121 and apply operable contact and pressure to hemorrhoid 128. One surface of the paddle ablation disk is designed to touch hemorrhoid 128 during treatment. The other side could also be designed to be used during treatment but it's not necessary. It is preferable to have the side designed to treat the hemorrhoid be flat, but it could also be arched or be different shapes to increase the contact area between the paddle ablation probe surface to the hemorrhoid. If the other side is not utilized for treatment, it could be designed to have a different shape and surface than the treatment side, for example, but limited to dome shape, triangular shape, etc. For one of the embodiments that utilizes RF power flow from the paddle ablation probe 53 to the hemorrhoid tissue to deliver heat, the surface that delivers energy is formed with electrically conductive and biocompatible materials, for example, but not limited to stainless steel and titanium. The rest of the body, including the side that is not designed to deliver energy, can be formed using biocompatible and non-conductive materials, for example, but not limited to various plastics such as polycarbonate, ABS, and nylons. For the other embodiment that utilizes resistive material to apply heat, the surface that delivers energy can be formed with biocompatible, thermally conductive, and electrically non-conductive materials.

The paddle ablation probe sheath 113 is attached to paddle ablation probe disk 112 and paddle ablation probe housing (handle)117. The diameter is narrower than the height of the paddle ablation probe disk 112. It is preferable to have the diameter as small as possible for better viewing of the treatment area during treatment, but the paddle ablation sheath 113 has to be sufficiently rigid for the user to apply operable contact pressure to the hemorrhoid during treatment. The outside surface of the paddle ablation probe sheath 113 is not necessarily touching hemorrhoids during treatment, but it is preferable to make the sheath bio-biocompatible.

The paddle ablation probe temperature sensor 114 is located inside of the paddle ablation probe disk 112. The temperature sensor 114 is used to monitor the maximum temperature reached at the ablation zone of the paddle ablation probe 53. For the safety of the ablation, it is ideal, not only to measure the temperature at the extent of the targeted hemorrhoid 128, but to also measure the energy source to control the heating of the tissue adjacent to the ablation zone. The temperature sensor 105 can be utilized using different temperature measuring modalities, for example, but not limited to thermocouples, thermistors, and RTDs.

One end of the paddle ablation probe temperature sensing cable 115 is attached tothe paddle ablation probe temperature sensing sensor 114. The other end of the temperature sensing cable is attached to the paddle ablation probe temperature sensor connector 120. The paddle ablation probe temperature sensing cable is positioned inside of the paddle ablation probe sheath 113 for the portion that extends along the paddle ablation probe sheath. If a thermocouple is used for temperature sensor 114, temperature sensing cable 115 and temperature sensor 114 is the same assembly of a thermocouple junction wire. If the temperature sensor is a thermistor or RTD, etc., the temperature sensing cable is a pair of electrical conductors insulated from each other.

One end of the paddle probe handle 117 is attached to the paddle probe sheath 113 and paddle probe insulator sheath 116. The other end of the paddle probe handle 117 is attached to the paddle probe temperature cable 115 and power cable 118. For the embodiment that utilizes RF energy for treatment, attachment of the paddle probe sheath 113 and paddle probe power cable 118 can be done inside of the paddle probe handle 117. The housing of the probe handle 117 can be formed with dielectric materials, for example, but not limited to polycarbonate, ABS, and Nylon, etc.

One end of the paddle ablation probe power cable 118 is connected to the paddle ablation probe power connector 119. The paddle ablation probe power cable 118 is located inside of the paddle ablation probe sheath 113 for the portion that extends along the paddle ablation probe sheath. For the embodiment of utilizing RF energy, the other end of the paddle ablation probe power connector 119 is connected to the paddle ablation probe sheath 113. The paddle ablation probe power cable 118 may be a single cable or use multiple conduction cables. In another embodiment utilizing resistive material 132 to deliver heat energy, one end of the paddle ablation probe power cable 118 is attached to the resistive material 132. The other end is attached to the paddle ablation probe power connector 119.

For another embodiment using conductive heat to deliver heat energy to hemorrhoid 128, resistive material 132 is utilized. The resistive material 132 is located at the inside wall of the paddle ablation probe disk 112. Several different materials can be used for resistive material 132, for example, but not limited to resistive wire, thermocouple wire, and semi-conductor materials. In an embodiment using thermocouple wire as resistive material, temperature sensor 114, resistive material 132, paddle ablation probe power cable 118, and paddle ablation probe temperature sensor cable 119 can be a single assembly of one thermocouple wire.

For an embodiment using RF energy to deliver heat energy and using paddle ablation probe sheath 113 to conduct electricity to the paddle ablation probe disk 112, paddle ablation probe insulation sheath 116 is utilized to prevent electrical conduction from the probe sheath 113 to surrounding tissues and/or to the user. The paddle ablation probe insulation sheath 116 is positioned outside of the paddle ablation probe sheath 113 along the length of the sheath. The paddle ablation probe insulation sheath can be formed using electrically insulating materials such as, but not limited to, mylar, PTFE, Nylon, and PVC, etc.

In an embodiment utilizing thermocouple wires as the paddle probe temperature sensor 114, temperature sensing connector 120 is a thermocouple connector that matches the compound of the thermocouple wire used. In an embodiment using a thermistor or RTD as temperature sensor 114, temperature sensing connector 120 is an electrical connector. One end of the thermocouple connector is attached to the temperature cable 115, and the other end plugs into the system 55.

One end of the ablation catheter power connector 119 is connected to the paddle power cable 118, and the other end of the power connector plugs into the system 55. The paddle power connector 119 has a portion that is electrically conductive that mates to electrically conductive material located inside of the system 55. The conductive material in the power connector 119 is preferably not exposed to the user. Outside housing of the power connector, 119 can be formed with dielectric materials, for example, but not limited to polycarbonate, ABS, and Nylon, etc.

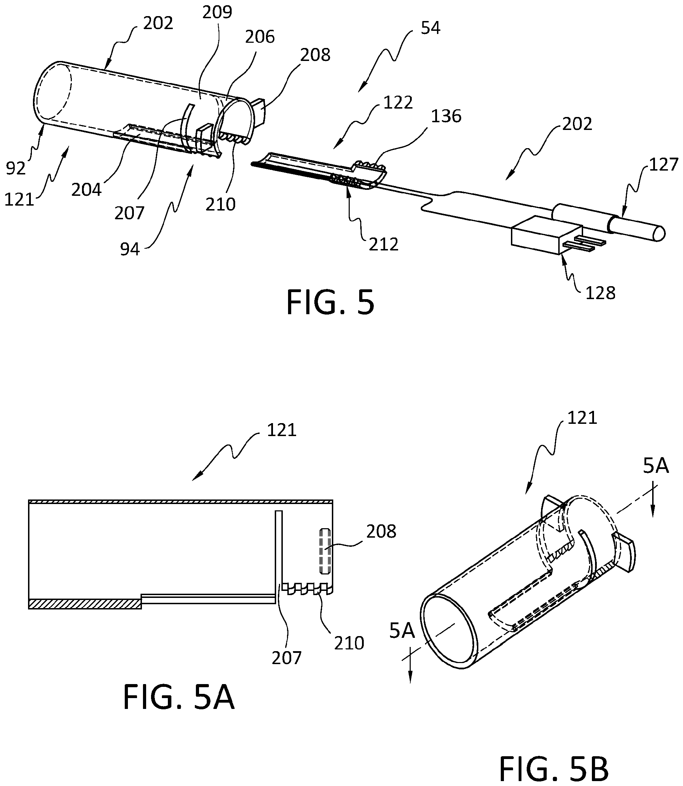

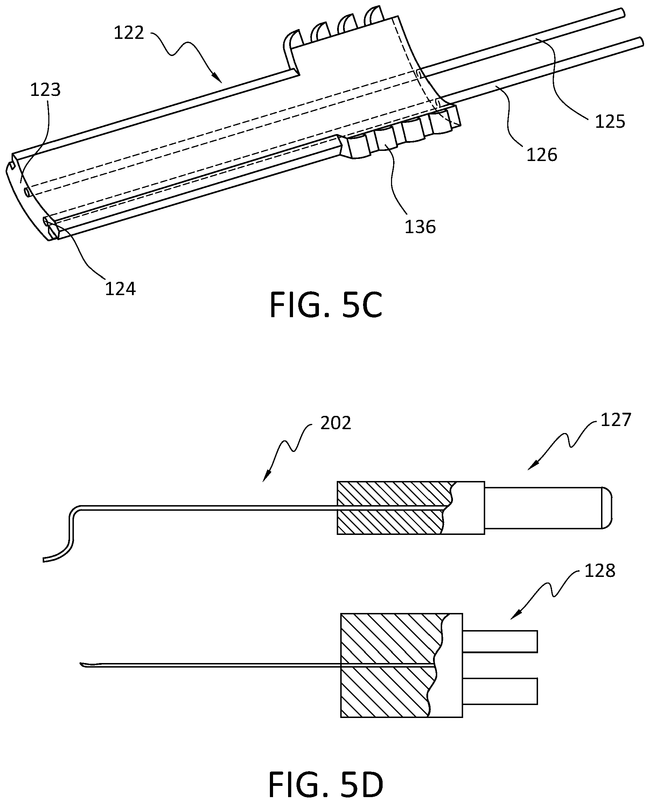

Referring to Figures 5, 5A, 5B, 5C, and 5D, an anoscope assembly 54 includes an elongated anoscope body 121 having a distal end 92 and a proximal end 94. The anoscope body includes a main portion 202 having a longitudinal slot 204 configured to function as a window opening to trap hemorrhoids. The proximal portion 206 depends proximally from the main portion 202. The proximal portion 206 has handles 208 thereon configured to be manipulated by a user so as to adjust the cross sectional area of the proximal portion 206. The anoscope assembly 54 includes an anoscope sliding element (window) 122, anoscope return path plate 123, anoscope temperature sensor 124, anoscope return path cable 125, anoscope temperature sensor cable 126, anoscope return path connector 127, and anoscope temperature sensor connector 128.

The sliding element 122 is configured to slide through the proximal portion 206 and through the longitudinal slot 204 when the cross sectional area of the proximal portion 206 is sufficiently increased by a user by manipulation of the handles, thus providing access and secure placement of the sliding element 122 within the longitudinal slot.

The proximal portion 206 defines a broken away ring type structure separated from the main portion by a cutaway radial slot 207. It remains integrally connected to the main portion 202 by integrally connecting portion 209.

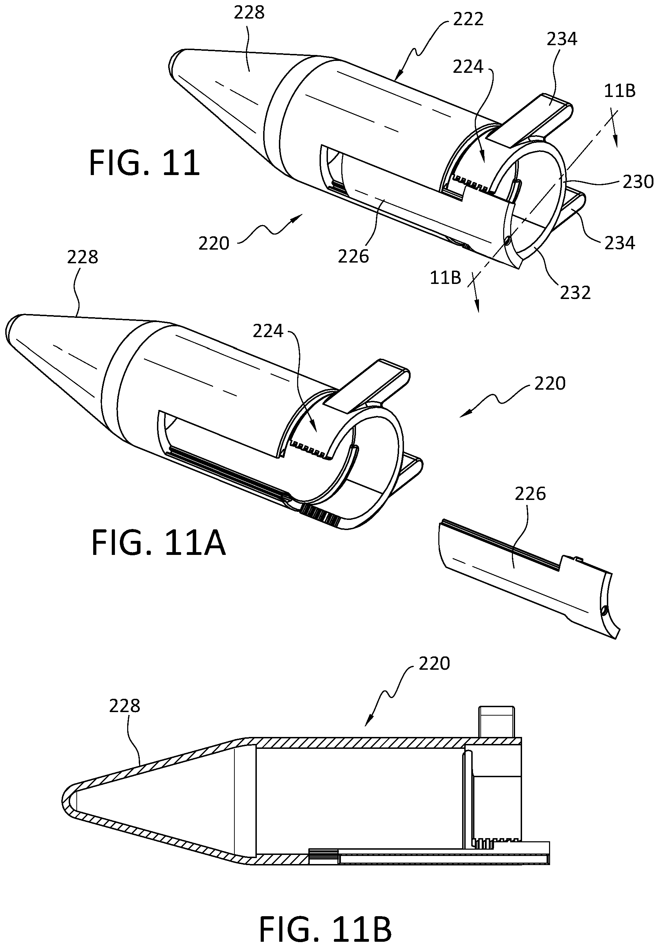

The anoscope body 121 is configured to be insertable into the human rectum. Thus, as described above, there is an opening on one side designed to trap hemorrhoids. The opening 204 is designed to fit and slide anoscope sliding element (window) 122. The anoscope body teeth 210 are configured to engage the anoscope sliding element teeth 136. The engagement of both teeth prevents the anoscope sliding window from backing out once it has pushed out distally. The anoscope body gap 133 is designed to make the area of the anoscope body where the anoscope body teeth is attached moveable outward when the anoscope sliding window is removed from the anoscope body. Anoscope handles 135 can be used to move the body where the anoscope body tooth is attached outward. In the preferred embodiments shown in Figure 5-5B (and in Figures 11-11B) the sliding element teeth 136 are angled and thus configured to be complementary operable with the proximal portion teeth 210 to provide insertion without manipulation of the handles. Manipulation of the handles is required to slide the sliding element in the reverse direction out from the proximal portion. The anoscope body is preferably but not necessarily made with transparent materials, for example, but not limited to polycarbonate, and acrylic, etc., for better viewing of the rectal wall for observation.

The length and diameter of the anoscope sliding window 122 are designed to fit the window opening of the anoscope 121.The anoscope sliding window is preferably but not necessarily made with transparent materials, for example, but not limited to polycarbonate, and acrylic, etc., for better viewing of the rectal wall for observation. Anoscope sliding window has an anoscope return path plate 123 attached to the distal end. Anoscope temperature sensor 124 is attached on or near the return path plate 123, where the temperature can be read tissue temperature at or adjacent to the return path plate 123. During ablation of hemorrhoid, the temperature read from the anoscope temperature sensor 124 may be the determining factor when to stop the procedure after completing it and/or interrupting it for safety. The temperature sensor 124 can be utilized using different temperature measuring modalities, for example, but not limited to thermocouples, thermistors, and RTDs. The anoscope temperature sensor cable 126 is attached to the anoscope temperature sensor 124. For the portion of the anoscope temperature sensor cable 126 that extends along the length of the anoscope sliding window 122, it is preferable but not limited to position the cable through the channel created inside of the sliding window. The other end of the anoscope temperature sensor cable 126 is attached to the anoscope temperature connector 128. In an embodiment that utilize thermocouples as the anoscope temperature sensor 124, the anoscope temperature cable 126 and anoscope temperature sensor 124 can be one assembly of thermocouple junction wire. The anoscope return path cable 125 is attached to the anoscope return path plate 123. For the portion of the anoscope return path cable 125 that extends along the length of the anoscope sliding window 122, it is preferable but not limited to position the cable through the channel created inside of the sliding window. The other end of the anoscope return path cable 125 is attached to the anoscope return path connector 127.

One end of the anoscope return path connector 127 is connected to the anoscope return path power cable 125, and the other end of the anoscope return path connector plugs into the system 55. The return path connector 127 has a portion that is electrically conductive that mates to electrically conductive material located inside of the system 55. The conductive material in the return path connector 127 is preferably not exposed to the user. Outside housing of the return path connector, 127 can be formed with dielectric materials, for example, but not limited to polycarbonate, ABS, and Nylon, etc.

In an embodiment of utilizing thermocouple wires as the return path temperature sensor 124, temperature sensing connector 128 is a thermocouple connector that matches the compound of the thermocouple wire used. In an embodiment of using a thermistor or RTD as temperature sensor 124, temperature sensing connector 128 is an electrical connector. One end of the temperature connector is attached to the temperature cable 126, and the other end plugs into the system 55.

For another embodiment of using resistive material 132 to deliver heat energy to hemorrhoid, the anoscope return path plate 123, the anoscope return path cable 125, and the anoscope return path connector 127 may not be necessary.

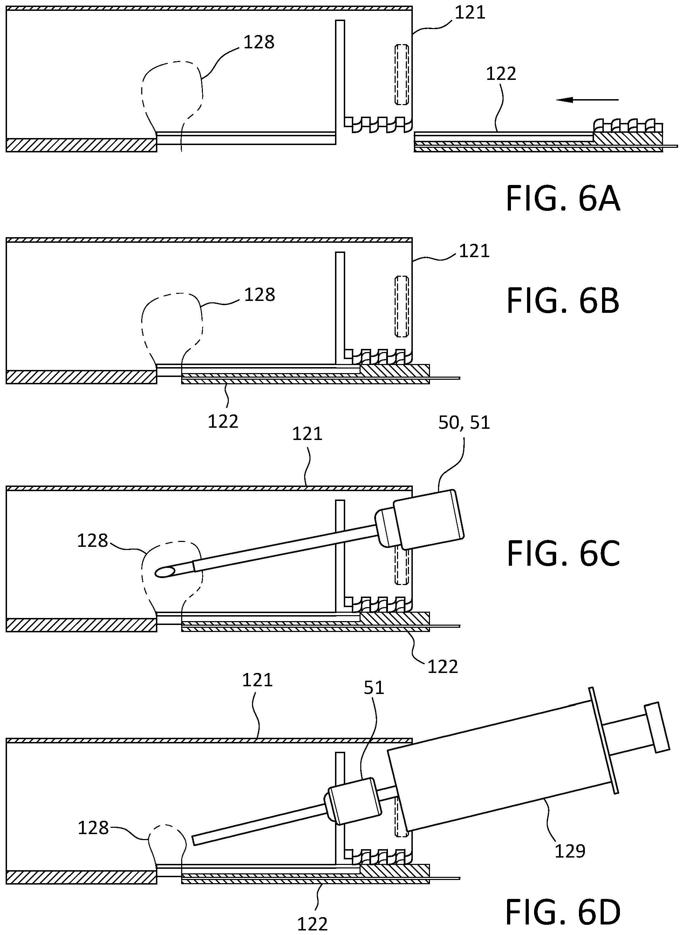

Referring to Figures 6A, 6B, 6C, 6D, 6E, 6F, and 6G, procedure steps using the ablation catheter 52 are illustrated. The steps include:
A) Insert anoscope body 121 into the rectum. For convenience, anoscope sliding element (window) 122 can be inserted into the window of the anoscope body 121 for better insertion into the rectum. Remove the anoscope sliding window 122 if inserted into the anoscope body 121. Position the hemorrhoid inside of the opening (window) of the anoscope body 121.
B) Slide the anoscope sliding window 122 into the window of the anoscope body 121 and trap hemorrhoid.
C) Mate dilator assembly 50 and dilator sheath assembly 51. Penetrate hemorrhoid.
D) Remove dilator assembly 50 from dilator sheath assembly 51 and attach syringe 129 to the dilator sheath. Remove trapped blood from the hemorrhoid using the syringe.
E) Remove syringe 129 from the dilator sheath and attach ablation catheter 52 to the dilator sheath. Ablate hemorrhoid.
F) Remove dilator sheath 51 and the ablation catheter 52.
G) Disengage the teeth 210 and 136 by moving the portion of the anoscope body 121 that is proximal to the user divided by the anoscope gap 207 by pulling the anoscope handles 208. Remove anoscope sliding window 122 from the anoscope body 121.

For another method of not using the dilator and removing trapped blood from hemorrhoid, after mating the ablation catheter 52 and dilator sheath 51, mated assembly of the ablation catheter and the dilator sheath can be penetrated inside of the hemorrhoid. Steps from C) through G) can be taken to proceed with the rest of the treatment.

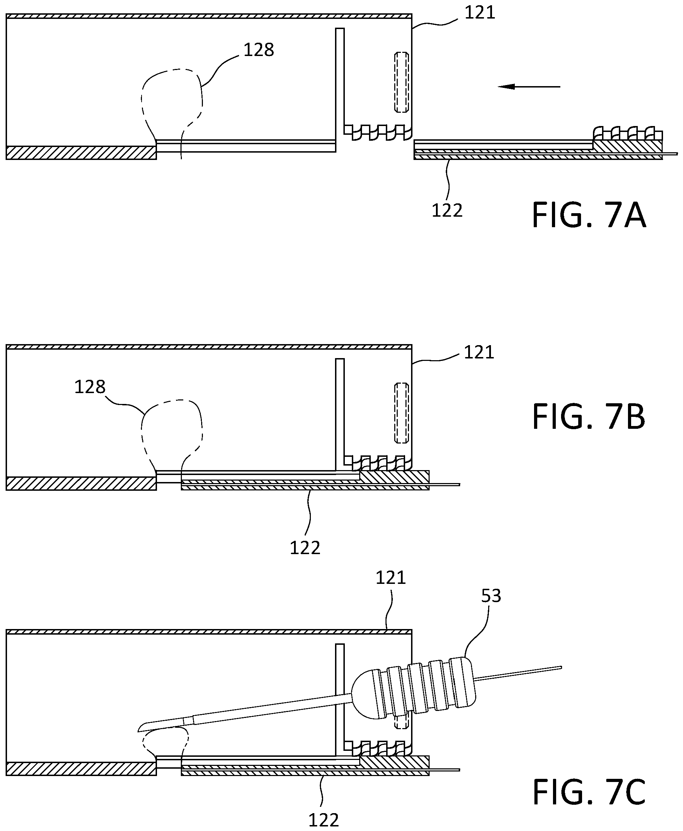

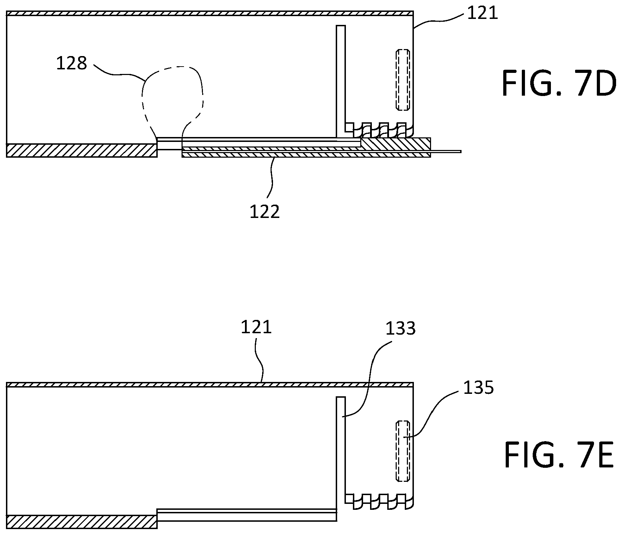

Referring to Figures 7A, 7B, 7C, 7D, and 7E, procedure steps using the paddle ablation probe assembly 53 are illustrated. The steps include:
A) Insert anoscope body 121 into the rectum. For convenience, anoscope sliding window 122 can be inserted into the window of the anoscope body for better insertion into the rectum. Remove the anoscope sliding window 122 if inserted into the anoscope body. Position the hemorrhoid inside of the window of the anoscope body 121.
B) Slide the anoscope sliding body 122 and trap hemorrhoid.
C) Touch hemorrhoid using paddle ablation probe assembly 53 and ablate hemorrhoid.
D) Remove the paddle probe. Disengage the teeth 210 and 136 by moving the portion of the anoscope body 121 that is proximal to the user divided by the anoscope gap 133 by pulling the anoscope handles 208. Remove anoscope sliding window 122 from the anoscope body 121.
E) Remove anoscope.

Referring to Figure 8, a system block diagram is illustrated of a preferred embodiment of the overall system for treating hemorrhoids, designated generally as 200. The system 200 uses an ablation control system assembly having microcontroller-based electronics capable of measuring different types of sensors as well as providing electric power to the ablation catheter and/or paddle probe. The power maybe, for example, RF power, DC power, or microwave power. System 55 includes ports to attach power output to the ablation catheter 52 or ablation paddle probe 53. The connection is made by attaching power connectors 110 or 119 to the system. System 55 also has a temperature sensing connection for the alation catheter temperature sensor 105 or paddle probe temperature sensor 114. The connection is made by attaching ablation catheter temperature connector 111 or the paddle probe temperature connector 120. It is preferable but not necessary to combine the power and temperature connectors into a one connector assembly both on the control system 55, and the catheter 52 / paddle probe 53 for ease of operation. System 55 also has a return path connection for the anoscope 54. The connection is made by attaching return path power connector 127 into the system. The system also has a connection to the return path temperature sensor by attaching a temperature sensor connector 128 into the system. The system also has a connector to attach ground pad 130. It is preferable but not necessary to combine the power and temperature connectors into one connector assembly both on the system 55, and the anoscope 54 for ease of operation.

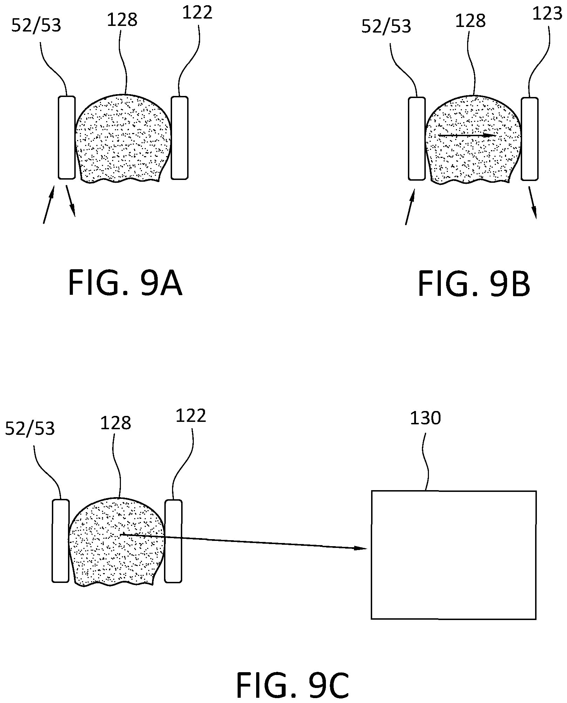

When ground pad 130 is attached, the system will sense the connection and it can automatically operate the system as a monopolar system described in Figure 9C. In this mode, the system will ignore the power connection between system 50 and the anoscope power connector 127 regardless of whether the connection is made or not.

When ground pad 130 is not attached, the system will sense the presence of the anoscope power connection 127 into the system. If the anoscope power connection is present, the system will operate the system as a bipolar system between the ablation catheter 52 or the paddle probe 53 and the anoscope return path pad. If the anoscope power connection is not present or the impedance between two poles described above is large at the time of the treatment, the system will operate the system as a resistive heating method and ignore the connection to the return path.

Ground pad 130 may or may not be needed based on the operating methods described in the following sections.

The user interface module assembly 140 is preferably a touch screen display used to communicate with the user in two ways. The screen displays the current status of the treatment as well as gives users valuable information during treatment, such as timer, temperature, power, voltage, current, or impedance information. It also provides user commands to the system by providing user clickable options. The system can also incorporate a treatment switch as a foot petal (not shown) and/or a button switch on the ablation catheter (or paddle probe) (not shown) to make it easier for the user to start and stop the procedure.

Referring to Figure 9A, 9B, and 9C, the diagram and current flow for several ablation methods are illustrated. The bar on the lefthand side represents ablation catheter 52 (or paddle probe 53). The circle in the middle represents a hemorrhoid 128. The bar on the right side represents the anoscope return path plate 122. The square represents ground pad 130.The arrows represent the current flow.

Referring to Figure 9A, the non-electrically conductive ablation method (i.e. resistive heating mode) is illustrated. The ablation catheter 52 (or paddle probe 53) are activated using resistive material 132. Therefore, there is no current flow from the catheter and probe 52/53 to hemorrhoid or the adjacent tissue. Conductive heat transfer is used to deliver heat to hemorrhoids. The return path plate 122 is not needed for the current path. Temperature measurement is the only function required from the return path. It is ideal to limit how high the catheter 52 and/or probe 53 can reach in terms of temperature in order to prevent over treatment of the tissue adjacent to the catheter and probe. In order to do that, ablation catheter temperature sensor 105 or paddle probe temperature sensor 114 is monitored to control the current going into the resistive material from the system 55. An ideal safeguard is to limit the maximum time that the current can flow to the resistive material by means of a maximum duty cycle and/or total treatment time limitation. The anoscope temperature sensor 124 located at or near the return path plate 123 is used to determine whether ablation is completed throughout the hemorrhoid. When the targeted temperature is reached, system 55 will shut off the treatment.

Referring to Figure 9B, a bipolar or return path plate mode is illustrated. The ablation catheter 52 (or paddle probe 53) are activated using RF or other methods that flow current from the source to the body. The current will flow from the ablation catheter 53 or paddle probe 53 to hemorrhoid 128, then to the return path plate 123. By minimizing the current flow distance from the catheter/probe to the return path plate, the energy requirement for treatment is relatively smaller compared to the method of using ground pad. In order to safeguard, it is ideal to limit the maximum time that the current can flow to hemorrhoids by means of maximum duty cycle and/or total treatment time limitation. It is also ideal to limit the maximum current that can flow into hemorrhoids. It can be achieved by impedance measurement of hemorrhoids and use a predetermined maximum current allowed to flow. The anoscope temperature sensor 124 located at or near the return path plate 123 is used to determine whether ablation is completed throughout hemorrhoid. When the targeted temperature is reached, system 55 will shut off the treatment.

Referring to Figure 9C, a monopolar mode is illustrated. The ablation catheter 52 (or paddle probe 53) are activated using RF or other methods that flow current from the source to the body. The current will flow from the ablation catheter 53 (or paddle probe 53) to hemorrhoid 128, then to the ground pad attached further away from the return path pad. One of the possible issues with the method illustrated in Figure 9B is that when the ablation and return path area are similar in size, usually the hottest area in terms of temperature is next to two poles. Since the temperature sensors are located near the two poles, it is possible that the two temperature sensors would present a temperature higher than the actual temperature of the hemorrhoid tissue; therefore, under treatment can occur. The method illustrated in Figure 9C eliminates this issue by placing the ground pad away from the anoscope temperature sensor which is relatively distanced from the targeted area. Temperature sensor 105 or 114 is activated to control the power output. As a safeguard, it is ideal to limit the maximum time that the current can flow to hemorrhoids by means of a maximum duty cycle and/or total treatment time limitation. It is also ideal to limit the maximum current that can flow into hemorrhoids. Although power control based on impedance measurement is feasible with monopolar systems, real-time impedance measurement and power control is less precise with monopolar systems due to higher impedance measurements due to the distance between the source and return path of the energy. Temperature sensor 124 is used as a safety feature not to increase the temperature at the anoscope temperature sensor 124 above a certain temperature. In this method, since the anoscope temperature sensor 124 is in the current path rather than at the end, the temperature measured would be highly reflective of the temperature of hemorrhoid.

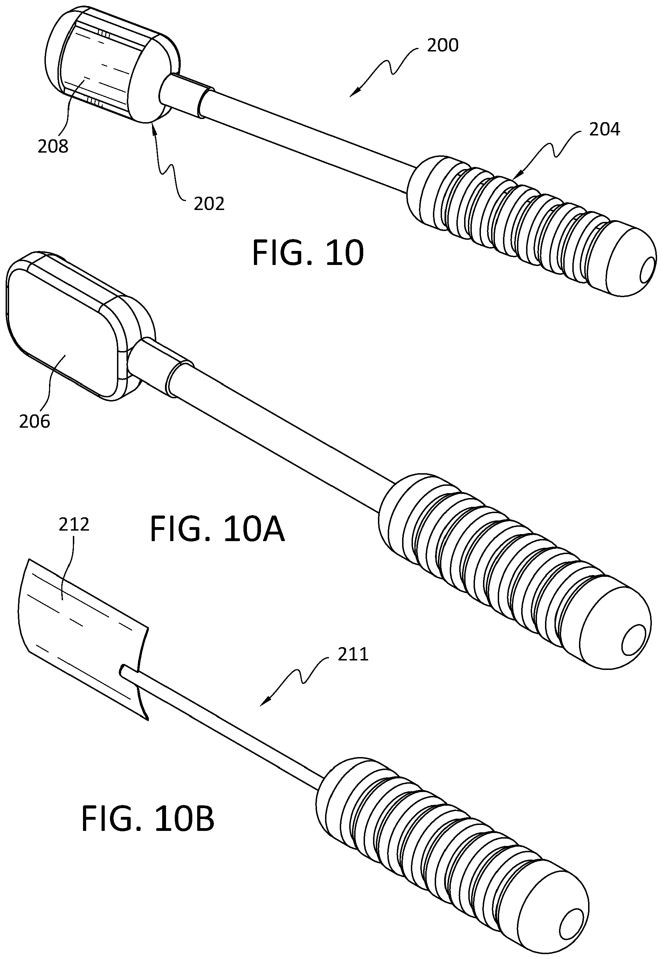

Referring now to Figures 10-10A, a first preferred embodiment of a treatment probe is illustrated, designated generally as 200, which includes a domed top ablation tip 202 on one end and a handle 204 on the other end. An ablation surface 206 opposes the dome shaped top surface 208.

Figure 10B illustrates a thin paddle probe, designated generally as 211.It includes an ablation surface 212 that can be a resistive or conductive surface. It can be active on both sides.

Referring now to Figures 11-11B a preferred second embodiment of an anoscope assembly is illustrated, designated generally as 220. This embodiment includes the same salient features as the

Figure 5 embodiment. It includes a main portion 222, proximal portion 224, and sliding element 226. The tip 228 is also shown in this illustration. The proximal portion 224 preferably has a relatively thin section 230 and a relatively thick section 232 to enhance flexibility. The ears 234 descend tangentially from the surface of the proximal portion 224 to provide ease in use.

The different methods to treat after trapping the hemorrhoid can be described as follows;
1. Invasive Method One
   a. Dilator and dilator sheath are place inside of the hemorrhoid.
   b. Dilator is retracted leaving the sheath inside of the hemorrhoid.
   c. Attach syringe on the sheath.
   d. Draw blood out of the hemorrhoid to reduce the mass of the hemorrhoid.
   e. Insert ablation probe into the sheath.
   f. Apply RF emerge. The ablation probe becomes first polarity. The ablation probe also has temperature sensor inside to control the power output.
   g. Return path (second polarity) is place on the sliding element of the anoscope assembly
   h. The sliding element also has temperature sensor to be used to determine when the procedure is done.
2. Invasive Method Two (Monopolar)
   a. Attach ground pad to the patients back or thigh.
   b. Dilator and dilator sheath are place inside of the hemorrhoid.
   c. Dilator is retracted leaving the sheath inside of the hemorrhoid.
   d. Attach syringe on the sheath.
   e. Draw blood out of the hemorrhoid to reduce the mass of the hemorrhoid.
   f. Insert ablation probe into the sheath.
   g. Apply RF emerge. The ablation probe becomes first polarity. The ablation probe also has temperature sensor inside to control the power output.
   h. The sliding has temperature sensor to be used to determine when the procedure is done.
3. Non-Invasive Method One (Conductive heating)
   a. Non-invasive conductive probe (paddle probe) is place on the top of the hemorrhoid.
   b. Probe has heater element and temperature monitoring element within the probe to control the energy applied to the hemorrhoid.
   c. The sliding element has temperature sensor to be used to determine when the procedure is done.
4. Non-Invasive Method Two (RF heating)
   a. Non-invasive RF probe is place on the top of the hemorrhoid.
   b. Apply RF emerge. The ablation probe becomes first polarity. The ablation probe also has temperature sensor inside to control the power output.
   c. Return path (second polarity) is place on the sliding element of the anoscope assembly.
   d. The sliding element also has temperature sensor to be used to determine when the procedure is done.
5. Non-Invasive Method Three (RF heating Monopolar)
   a. Attach ground pad to the patients back or thigh.
   b. Non-invasive RF probe is place on the top of the hemorrhoid.
   c. Apply RF emerge. The ablation probe becomes first polarity. The ablation probe also has temperature sensor inside to control the power output.
   d. The sliding has temperature sensor to be used to determine when the procedure is done

It is to be understood that although the invention has been described above in terms of particular embodiments, the foregoing embodiments are provided as illustrative only, and do not limit or define the scope of the invention. Various other embodiments, including but not limited to the following, are also within the scope of the claims. For example, elements and components described herein may be further divided into additional components or joined together to form fewer components for performing the same functions.

Any of the functions disclosed herein may be implemented using means for performing those functions. Such means include, but are not limited to, any of the components disclosed herein, such as the computer-related components described below.

The techniques described above may be implemented, for example, in hardware, one or more computer programs tangibly stored on one or more computer-readable media, firmware, or any combination thereof. The techniques described above may be implemented in one or more computer programs executing on (or executable by) a programmable computer including any combination of any number of the following: a processor, a storage medium readable and/or writable by the processor (including, for example, volatile and non- volatile memory and/or storage elements), an input device, and an output device. Program code may be applied to input entered using the input device to perform the functions described and to generate output using the output device.

Embodiments of the present invention include features which are only possible and/or feasible to implement with the use of one or more computers, computer processors, and/or other elements of a computer system. Such features are either impossible or impractical to implement mentally and/or manually. For example, embodiments of the present invention may read and write data to electronic memory devices (such as RFID tags) and/or to distributed ledgers (such as a blockchain), which are functions that cannot be performed mentally or manually.

Any claims herein which affirmatively require a computer, a processor, a memory, or similar computer-related elements, are intended to require such elements, and should not be interpreted as if such elements are not present in or required by such claims. Such claims are not intended, and should not be interpreted, to cover methods and/or systems which lack the recited computer-related elements. For example, any method claim herein which recites that the claimed method is performed by a computer, a processor, a memory, and/or similar computer-related element, is intended to, and should only be interpreted to, encompass methods which are performed by the recited computer-related element(s). Such a method claim should not be interpreted, for example, to encompass a method that is performed mentally or by hand (e.g., using pencil and paper). Similarly, any product claim herein which recites that the claimed product includes a computer, a processor, a memory, and/or similar computer-related element, is intended to, and should only be interpreted to, encompass products which include the recited computer-related element(s). Such a product claim should not be interpreted, for example, to encompass a product that does not include the recited computer-related element(s).

Each computer program within the scope of the claims below may be implemented in any programming language, such as assembly language, machine language, a high-level procedural programming language, or an object-oriented programming language. The programming language may, for example, be a compiled or interpreted programming language.

Each such computer program may be implemented in a computer program product tangibly embodied in a machine-readable storage device for execution by a computer processor. Method steps of the invention may be performed by one or more computer processors executing a program tangibly embodied on a computer-readable medium to perform functions of the invention by operating on input and generating output. Suitable processors include, by way of example, both general and special purpose microprocessors. Generally, the processor receives (reads) instructions and data from a memory (such as a read-only memory and/or a random access memory) and writes (stores) instructions and data to the memory. Storage devices suitable for tangibly embodying computer program instructions and data include, for example, all forms of non-volatile memory, such as semiconductor memory devices, including EPROM, EEPROM, and flash memory devices; magnetic disks such as internal hard disks and removable disks; magneto- optical disks; and CD-ROMs. Any of the foregoing may be supplemented by, or incorporated in, specially-designed ASICs (application-specific integrated circuits) or FPGAs (Field- Programmable Gate Arrays). A computer can generally also receive (read) programs and data from, and write (store) programs and data to, a non-transitory computer-readable storage medium such as an internal disk (not shown) or a removable disk. These elements will also be found in a conventional desktop or workstation computer as well as other computers suitable for executing computer programs implementing the methods described herein, which may be used in conjunction with any digital print engine or marking engine, display monitor, or other raster output device capable of producing color or gray scale pixels on paper, film, display screen, or other output medium.

Any data disclosed herein may be implemented, for example, in one or more data structures tangibly stored on a non-transitory computer-readable medium. Embodiments of the invention may store such data in such data structure(s) and read such data from such data structure(s).
Any step or act disclosed herein as being performed, or capable of being performed, by a computer or other machine, may be performed automatically by a computer or other machine, whether or not explicitly disclosed as such herein. A step or act that is performed automatically is performed solely by a computer or other machine, without human intervention. A step or act that is performed automatically may, for example, operate solely on inputs received from a computer or other machine, and not from a human. A step or act that is performed automatically may, for example, be initiated by a signal received from a computer or other machine, and not from a human. A step or act that is performed automatically may, for example, provide output to a computer or other machine, and not to a human.

## Claims

1. A system (55) for treating hemorrhoids, wherein the system comprises:
a) an anoscope assembly (54), comprising:
i) an elongated anoscope body (121) having a distal end (92) and a proximal end (94), said anoscope body (121) being configured to be insertable into the rectum of a patient, said elongated anoscope body (121), comprising:
1. a main portion (202) having a longitudinal slot (204) configured to function as a window opening to trap hemorrhoids;
2. a proximal portion (206) extending proximally from said main portion (202), said proximal portion (206) having handles (208) thereon configured to be manipulated by a user so as to adjust the cross-sectional area of the proximal portion (206);
ii) a sliding element (122) configured to slide through the proximal portion (206) and through said longitudinal slot (204) when the cross-sectional area of the proximal portion (206) is sufficiently increased by a user by manipulation of said handles (208), thus providing access and secure placement of the sliding element (122) within the longitudinal slot (204); and,
b) a treatment probe operably connected to said anoscope assembly (54), configured to provide energy to a hemorrhoid during treatment;
**characterized in that**
said proximal portion (206) defines a broken away ring type structure, said broken away ring type structure separates from the main portion (202) by a cutaway radial slot (207), and remains integrally connected to the main portion (202) by an integrally connecting portion (209).

2. The system (55) of Claim 1 further comprising an ablation control system assembly operatively connectable to said anoscope assembly (54).

3. The system (55) of Claim 1 wherein said elongated anoscope body (121) and said sliding element (122) are configured to provide access and placement of the sliding element (122) without manipulation of the handles (208) when the sliding element (122) is being slid into the proximal portion (206), but manipulation of the handles (208) is required to slide the sliding element (122) in the reverse direction out from the proximal portion (206).

4. The system (55) of Claim 1 wherein said elongated anoscope body (121) and said sliding element (122) are configured to provide access and placement of the sliding element (122) without manipulation of the handles (208) when the sliding element (122) is being slid into the proximal portion (206), but manipulation of the handles (208) is required to slide the sliding element (122) in the reverse direction out from the proximal portion (206); and, wherein:
a) the broken away ring type structure having proximal portion teeth (210) on broken away edges thereof; and
b) said sliding element (122) includes a sliding element proximal portion having sliding element teeth (136) configured to be complementary operable with said proximal portion teeth (210) to provide insertion without manipulation of the handles (208), but manipulation of the handles (208) is required to slide the sliding element (122) in the reverse direction out from the proximal portion (206).

5. The system (55) of Claim 1, wherein:
a) the broken away ring type structure having proximal portion teeth (210) on broken away edges thereof; and,
b) said sliding element (122) includes a sliding element proximal portion having sliding element teeth (136) configured to be complementary operable with said proximal portion teeth (210) to prevent relative movement therebetween.

6. The system (55) of Claim 5, wherein said broken away ring type structure is integrally connected to said main portion (202).

7. The system (55) of Claim 5, wherein said handles (208) comprise a pair of handles (208) extending from said proximal portion (206).

## Patentansprüche

1. System (55) zum Behandeln von Hämorrhoiden, wobei das System umfasst:
a) eine Anoskopbaugruppe (54), umfassend:
i) einen länglichen Anoskopkörper (121), der ein distales Ende (92) und ein proximales Ende (94) aufweist, wobei der Anoskopkörper (121) konfiguriert ist, um in das Rektum eines Patienten eingeführt werden zu können, der längliche Anoskopkörper (121) umfassend:
1. einen Hauptabschnitt (202), der einen Längsschlitz (204) aufweist, der konfiguriert ist, um als eine Fensteröffnung zu fungieren, um Hämorrhoiden einzufangen;
2. einen proximalen Abschnitt (206), der sich von dem Hauptabschnitt (202) proximal erstreckt, wobei der proximale Abschnitt (206) Griffe (208) daran aufweist, die konfiguriert sind, um durch einen Benutzer bedient zu werden, um die Querschnittsfläche des proximalen Abschnitts (206) anzupassen;
ii) ein Gleitelement (122), das konfiguriert ist, um durch den proximalen Abschnitt (206) und durch den Längsschlitz (204) zu gleiten, wenn die Querschnittsfläche des proximalen Abschnitts (206) durch einen Benutzer durch Bedienung der Griffe (208) ausreichend vergrößert wird, wodurch Zugang und eine sichere Platzierung des Gleitelements (122) innerhalb des Längsschlitzes (204) bereitgestellt werden; und,
b) eine Behandlungssonde, die mit der Anoskopbaugruppe (54) wirkverbunden ist, die konfiguriert ist, um während der Behandlung Energie an eine Hämorrhoide bereitzustellen;
**dadurch gekennzeichnet, dass**
der proximale Abschnitt (206) eine weggebrochene ringartige Struktur definiert, sich die weggebrochene ringartige Struktur durch einen ausgeschnittenen radialen Schlitz (207) von dem Hauptabschnitt (202) trennt und durch einen einstückigen Verbindungsabschnitt (209) mit dem Hauptabschnitt (202) einstückig verbunden bleibt.

2. System (55) nach Anspruch 1, ferner umfassend eine Ablationssteuersystembaugruppe, die mit der Anoskopbaugruppe (54) wirkverbunden werden kann.

3. System (55) nach Anspruch 1, wobei der längliche Anoskopkörper (121) und das Gleitelement (122) konfiguriert sind, um Zugriff auf und Platzierung des Gleitelements (122) ohne Bedienung der Griffe (208) bereitzustellen, wenn das Gleitelement (122) in den proximalen Abschnitt (206) gleitet, aber eine Bedienung der Griffe (208) erforderlich ist, damit das Gleitelement (122) in die entgegengesetzte Richtung aus dem proximalen Abschnitt (206) herausgleitet.

4. System (55) nach Anspruch 1, wobei der längliche Anoskopkörper (121) und das Gleitelement (122) konfiguriert sind, um Zugriff auf und Platzierung des Gleitelements (122) ohne Bedienung der Griffe (208) bereitzustellen, wenn das Gleitelement (122) in den proximalen Abschnitt (206) gleitet, aber eine Bedienung der Griffe (208) erforderlich ist, damit das Gleitelement (122) in die entgegengesetzte Richtung aus dem proximalen Abschnitt (206) herausgleitet; und wobei:
a) die weggebrochene ringartige Struktur Zähne (210) in dem proximalen Abschnitt an weggebrochenen Kanten davon aufweist; und
b) das Gleitelement (122) einen proximalen Gleitelementabschnitt einschließt, der Gleitelementzähne (136) aufweist, die konfiguriert sind, um komplementär zu den Zähnen (210) des proximalen Abschnitts funktionsfähig zu sein, um eine Einführung ohne Bedienung der Griffe (208) bereitzustellen, aber eine Bedienung der Griffe (208) erforderlich ist, damit das Gleitelement (122) in die entgegengesetzte Richtung aus dem proximalen Abschnitt (206) herausgleitet.

5. System (55) nach Anspruch 1, wobei:
a) die weggebrochene ringartige Struktur Zähne (210) in dem proximalen Abschnitt an weggebrochenen Kanten davon aufweist; und,
b) das Gleitelement (122) einen proximalen Gleitelementabschnitt einschließt, der Gleitelementzähne (136) aufweist, die konfiguriert sind, um komplementär zu den Zähnen (210) des proximalen Abschnitts funktionsfähig zu sein, um eine Relativbewegung zwischen ihnen zu verhindern.

6. System (55) nach Anspruch 5, wobei die weggebrochene ringartige Struktur mit dem Hauptabschnitt (202) einstückig verbunden ist.

7. System (55) nach Anspruch 5, wobei die Griffe (208) ein Paar Griffe (208) umfassen, die sich von dem proximalen Abschnitt (206) erstrecken.

## Revendications

1. Système (55) de traitement des hémorroïdes, dans lequel le système comprend :
a) un ensemble d'anuscope (54), comprenant :
i) un corps d'anuscope allongé (121) ayant une extrémité distale (92) et une extrémité proximale (94), ledit corps d'anuscope (121) étant conçu pour être inséré dans le rectum d'un patient, ledit corps d'anuscope allongé (121), comprenant :
1. une partie principale (202) ayant une fente longitudinale (204) conçue pour fonctionner comme une ouverture de fenêtre pour piéger les hémorroïdes ;
2. une partie proximale (206) s'étendant proximalement à partir de ladite partie principale (202), ladite partie proximale (206) ayant des poignées (208) conçues sur celle-ci pour être manipulées par un utilisateur de manière à ajuster la zone de section transversale de la partie proximale (206) ;
ii) un élément coulissant (122) conçu pour glisser à travers la partie proximale (206) et à travers ladite fente longitudinale (204), lorsque la section transversale de la partie proximale (206) est suffisamment augmentée par un utilisateur par la manipulation desdites poignées (208), permettant ainsi l'accès et le placement sécurisé de l'élément coulissant (122) à l'intérieur de la fente longitudinale (204) ; et,
b) une sonde de traitement reliée de manière opérationnelle à l'ensemble d'anuscope (54), configurée pour fournir de l'énergie à une hémorroïde pendant le traitement ;
**caractérisé en ce que**
ladite partie proximale (206) définit une structure de type anneau brisé, ladite structure de type anneau brisé se sépare de la partie principale (202) par une fente radiale découpée (207), et reste intégralement reliée à la partie principale (202) par une partie de liaison intégrale (209).

2. Système (55) selon la revendication 1, comprenant en outre un ensemble de système de commande d'ablation pouvant être connecté de manière opérationnelle à cet ensemble d'anuscope (54).

3. Système (55) selon la revendication 1, dans lequel ledit corps d'anuscope allongé (121) et ledit élément coulissant (122) sont conçus pour permettre l'accès et le placement de l'élément coulissant (122) sans manipulation des poignées (208), lorsque l'élément coulissant (122) est glissé dans la partie proximale (206), mais la manipulation des poignées (208) est nécessaire pour faire glisser l'élément coulissant (122) dans la direction inverse pour le faire sortir de la partie proximale (206).

4. Système (55) selon la revendication 1, dans lequel ledit corps d'anuscope allongé (121) et ledit élément coulissant (122) sont conçus pour permettre l'accès et le placement de l'élément coulissant (122) sans manipulation des poignées (208), lorsque l'élément coulissant (122) est glissé dans la partie proximale (206), mais la manipulation des poignées (208) est nécessaire pour faire glisser l'élément coulissant (122) dans la direction inverse hors de la partie proximale (206) ; et, dans lequel :
a) la structure de type anneau brisé ayant des dents de partie proximale (210) sur ses bords brisés ; et
b) ledit élément coulissant (122) comporte une partie proximale d'élément coulissant ayant des dents d'élément coulissant (136) conçues pour être complémentaires des dents de partie proximale (210) afin de permettre l'insertion sans manipulation des poignées (208), mais la manipulation des poignées (208) est nécessaire pour faire glisser l'élément coulissant (122) dans le sens inverse à partir de la partie proximale (206).

5. Système (55) selon la revendication 1, dans lequel :
a) la structure de type anneau brisé ayant des dents de partie proximale (210) sur ses bords brisés ; et,
b) ledit élément coulissant (122) comporte une partie proximale de l'élément coulissant ayant des dents d'élément coulissant (136) conçues pour être complémentaires des dents de partie proximale (210) afin d'empêcher tout mouvement relatif entre elles.

6. Système (55) selon la revendication 5, dans lequel ladite structure de type anneau brisé est intégralement reliée à ladite partie principale (202).

7. Système (55) selon la revendication 5, dans lequel lesdites poignées (208) comprennent une paire de poignées (208) s'étendant à partir de ladite partie proximale (206).
